# EUROPEAN PATENT APPLICATION

(11) **EP 1 518 515 A1**
(43) Date of publication of application: **30.03.2005**
(21) Application number: 04255205.9
(22) Date of filing: 27.08.2004
(51) Int. Cl.: A61F 2/01, A61F 2/06

(54) **Self-expanding stent and stent delivery system for treatment of vascular stenosis**

(30) Priority: 29.08.2003 US 651569
(71) Applicant: Cordis Neurovascular, Inc., Miami Lakes, Florida 33014 (US)
(72) Inventor: Jones, Donald K., Lauderhill Florida 33351 (US); Mitelberg, Vladimir, Aventura Florida 33180 (US)
(74) Representative: Mercer, Christopher Paul

(57) **Abstract**

A self-expanding stent and stent delivery system are provided for treating vascular diseases such as partially occluded blood vessels within the brain. The self-expanding stent (20) is preferably mounted on an elongated core wire including proximal, intermediate and distal cylindrical members disposed about the core wire. The proximal, intermediate, and distal cylindrical members are spaced apart such that first and second gaps are formed. The stent, which includes anchor members, is mounted on the intermediate cylindrical member such that the anchor members (52) interlock within the gaps between the cylindrical members. The self-expanding stent and elongated core wire disposed within a delivery lumen (7) of a balloon catheter (2), such that the balloon catheter compresses and constrains the stent about the intermediate cylindrical member to thereby interlock the stent onto the core wire. The elongated core wire further includes an expandable capture basket (24) attached to the distal end of the elongated core wire in order to capture embolic debris released during angioplasty and during the deployment of the self-expanding stent.

## Description

### Background of the Invention

### Field of the Invention

This invention relates to intravascular stents, stent delivery systems, and methods of treating a stenosis within a blood vessel. More specifically, this invention relates to self-expanding stents with integral balloon catheters which may be used for percutaneous transluminal angioplasty of occluded blood vessels within the brain of a patient.

### Description of the Prior Art

On a worldwide basis, nearly one million balloon angioplasties are performed annually to treat vascular diseases such as blood vessels that are clogged or narrowed by a lesion or stenosis. The objective of this procedure is to increase the inner diameter of the partially occluded blood vessel lumen. In an effort to prevent restenosis without requiring surgery, short flexible cylinders or scaffolds, referred to as stents, are often placed into the blood vessel at the site of the stenosis.

Stents are typically made of metal or polymers and are widely used for reinforcing diseased blood vessels. Some stents are expanded to their proper size using a balloon catheter. Such stents are referred to as "balloon expandable" stents. Other stents, referred to as "self-expanding" stents, are designed to elastically resist compression in a self-expanding manner. Balloon expandable stents and self-expanding stents are compressed into a small diameter cylindrical form and deployed within a blood vessel using a catheter-based delivery system, such as a balloon catheter.

Several balloon catheters have been disclosed in prior patents. One such balloon catheter is disclosed in U.S. Patent No. 5,843,090, entitled "Stent Delivery Device," wherein a balloon catheter, having inner and outer catheters, with the outer catheter having a second lumen for inflation of a balloon, is used as a stent delivery device. U.S. Patent No. 5,639,274, entitled "Integrated Catheter System for Balloon Angioplasty and Stent Delivery," discloses an integrated catheter system including a stent catheter and a balloon angioplasty catheter, where the stent catheter contains a stent and is displaced over the balloon catheter. However, current balloon catheters are typically too large and inflexible to traverse the tortuous blood vessels within the brain.

Recently, filters mounted on the distal end of guidewires have been proposed for intravascular blood filtration during balloon angioplasty and the delivery of vascular stents. One such filter is disclosed in U.S. Patent No. 6,168,579, entitled "Filter Flush System and Methods of Use." This patent discloses a filter flush system for temporary placement of a filter in a blood vessel. The filter system includes a guidewire having an expandable filter which may be collapsed to pass through the lumen of a guiding catheter and may then be expanded upstream of a stenosis prior to angioplasty or to the placement of a stent. U.S. Patent Application Publication No. 2002/0115942, entitled "Low Profile Emboli Capture Device," discloses an emboli capture device comprised of a filter and a self-expanding stent. The self-expanding stent is attached to the filter in order to open the filter when the emboli capture device is placed within an artery.

### Summary of the Invention

In accordance with the present invention, there is provided a self-expanding stent and stent delivery system. The stent delivery system includes a balloon catheter comprised of an elongated catheter having a delivery lumen and an inflation lumen. Mounted on the distal section of the elongated catheter is an inflatable balloon which communicates with the inflation lumen. Disposed within the delivery lumen of the elongated catheter is an elongated core member. The elongated core member includes a proximal cylindrical member and a distal cylindrical member, both disposed about the distal portion of the core member. The distal cylindrical member is generally positioned distally of the proximal cylindrical member and spaced apart from the proximal cylindrical member to define a gap having a predetermined length. The self-expanding stent is comprised of a small diameter skeletal tubular member having a thin wall. The wall of the skeletal tubular member is comprised of a plurality of cells which are formed by a plurality of interconnected strut members. A cylindrical anchor member is placed on one of the strut members. The cylindrical anchor member has a length less than the length of the gap between the proximal cylindrical member and the distal cylindrical member. The self-expanding stent is mounted on one of the cylindrical members and is aligned such that the cylindrical anchor member is interlocked within the gap between the proximal cylindrical member and the distal cylindrical member to thereby retain the stent on the elongated core member.

In accordance with another aspect of the present invention, the elongated core member is comprised of a wire. In addition, the skeletal tubular member includes threads formed on one of the strut members, and the cylindrical anchor member takes the form of a helically wound coil, preferably formed of radiopaque material, wound onto the threads on the strut member. Additionally, the self-expanding stent includes eight cylindrical anchor members, each cylindrical anchor member taking the form of a helically wound coil formed of radiopaque material, being wound onto threads formed on each of eight strut members.

In accordance with yet another aspect of the present invention, there is provided a self-expanding stent and stent delivery system including a balloon catheter comprised of an elongated catheter having a delivery lumen. The balloon catheter includes an expandable balloon mounted on the distal section of the elongated catheter. An elongated core member is slidably disposed within the delivery lumen of the elongated catheter. A stop member extends radially outward from the core member, and a self-expanding stent is mounted on the elongated core member engaging the stop member so that the stent can be moved through the delivery lumen when the elongated core member is moved through the delivery lumen.

In accordance with another aspect of the present invention, the balloon catheter includes an inflation lumen communicating with the expandable balloon. The elongated core member takes the form of a wire. Furthermore, the stop member is comprised of a cylindrical coil disposed about the elongated core member. In this case, the self-expanding stent is comprised of a small diameter skeletal tubular member having a thin wall. The wall of the skeletal tubular member includes a plurality of cells which are formed by a plurality of interconnected strut members with a cylindrical anchor member being disposed about one of the strut members.

In accordance with a further aspect of the present invention, there is provided a method of treating a stenosis including the steps of inserting a balloon catheter into a vessel of a patient, advancing the balloon catheter until the balloon catheter is positioned across a stenosis within the vessel, inserting a self-expanding stent mounted on an elongated core member into the delivery lumen of the catheter and advancing the self-expanding stent and elongated core member distally through the delivery lumen until the self-expanding stent is aligned approximate the stenosis. The method further includes the steps of injecting a fluid into the inflation lumen of the balloon catheter to thereby inflate the balloon, removing the fluid from within the inflation lumen to thereby deflate the balloon, and withdrawing the catheter proximally, allowing the self-expanding stent to expand within the vessel and thus disengaging the self-expanding stent from the elongated core member. Finally, the method includes the step of withdrawing the balloon catheter and elongated core member from the vessel of the patient.

In accordance with still another aspect of the present invention, there is provided a method of treating a stenosis comprising the steps of inserting a balloon catheter into a blood vessel of a patient over a guidewire, advancing the guidewire and the balloon catheter until the balloon catheter is positioned across a stenosis within the blood vessel, and removing the guidewire. The method further includes the steps of inserting a self-expanding stent mounted on an elongated core member into the delivery lumen of the catheter and advancing the self-expanding stent and elongated core member distally through the delivery lumen until the self-expanding stent is aligned approximate the stenosis. Then, a fluid is injected into the inflation lumen of the catheter to thereby inflate the balloon. The method further includes the steps of removing the fluid from within the inflation lumen to thereby deflate the balloon, withdrawing the catheter proximally, allowing the self-expanding stent to expand within the blood vessel and releasing the cylindrical anchor member from the gap to thereby disengage the self-expanding stent from the core member, and withdrawing the catheter and the core member from the blood vessel of the patient.

### Brief Description of the Drawings

Figure 1 is a partially sectioned view of a balloon catheter containing a self-expanding stent mounted on an elongated core wire, said wire including a capture basket attached to its distal end;
Figure 2 is a sectioned view of the elongated core wire of Figure 1 having a self-expanding stent mounted on the elongated core wire and having a capture basket attached to the distal end of the core wire;
Figure 3 is a sectioned view of the balloon catheter of Figure 1 within a blood vessel;
Figure 3a is a sectioned view of the balloon catheter of Figure 1 within a blood vessel and having the capture basket deployed and expanded within the blood vessel;
Figure 4 is a sectioned view of the balloon catheter, within the blood vessel, having the balloon fully expanded;
Figure 5 is a sectioned view of the balloon catheter being moved proximally thereby allowing the self-expanding stent to begin expanding within the blood vessel;
Figure 6 is a sectioned view of the self-expanding stent fully expanded within the blood vessel while the elongated core wire remains extended through the stent;
Figure 7 is a sectioned view of the balloon catheter advanced distally over the core wire and through the self-expanding stent;
Figure 8 is a sectioned view of the balloon catheter and elongated core wire withdrawn proximally through the self-expanding stent; and,
Figure 9 is a view of the self-expanding stent within the blood vessel with the balloon catheter and elongated core wire removed from within the blood vessel.

### Description of the Preferred Embodiment

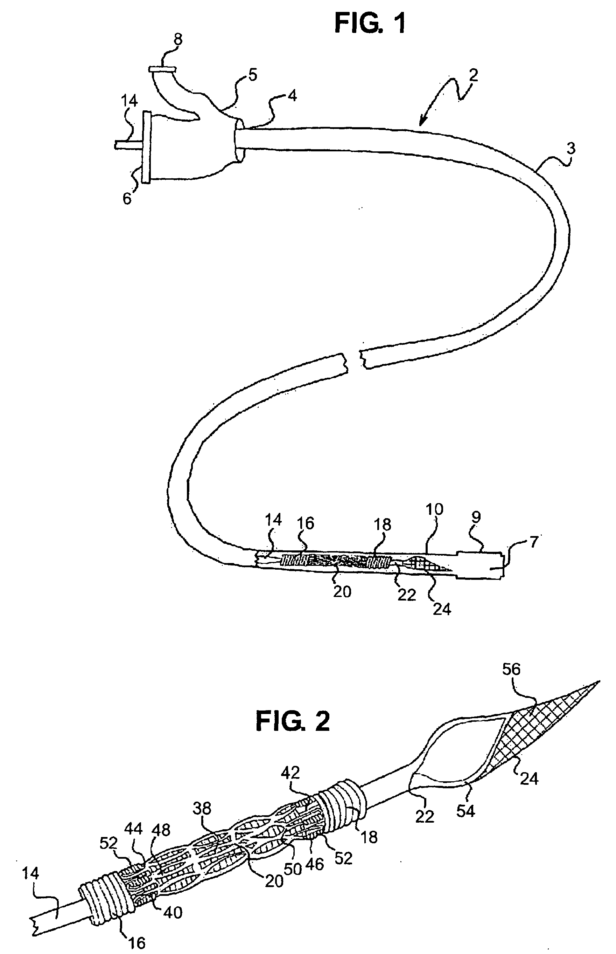

Figure 1 illustrates a balloon catheter 2 comprising an elongated outer catheter 3. Attached to the proximal end 4 of the outer catheter 3 is a coupling member 5. The coupling member 5 includes a delivery port 6 which communicates with a delivery lumen 7, which extends throughout the length of the balloon catheter 2. The coupling member 5 also includes an activation port 8 used to activate and expand an expandable balloon 9 disposed about the distal portion 10 of the outer catheter 3. The balloon catheter 2 should be rigid enough to be pushed distally through a blood vessel, yet flexible enough to traverse the narrow and tortuous blood vessels within the brain.

Slidably disposed within the delivery lumen 7 is an elongated core member 14, preferably taking the form of an elongated core wire. Disposed about the elongated core wire 14 are a proximal cylindrical member 16 and a distal cylindrical member 18. A self-expanding stent 20 is mounted on the elongated core wire 14. The proximal and distal cylindrical members 16,18 serve as stop members extending radially outward from the core wire 14 to engage the stent 20 with the elongated core wire such that the stent can be moved proximally and distally through the delivery lumen 7. Attached to the distal end 22 of the elongated core wire 14 is an expandable capture basket 24.

Figure 2 illustrates the self-expanding stent 20 mounted on the elongated core wire 14. Disposed about the elongated core wire 14 is a proximal cylindrical member 16. Preferably, the proximal cylindrical member 16 is a helically wound flexible coil made of metal, but may alternatively be formed of a polymer material. An intermediate cylindrical member 38 (shown within the stent) is also disposed about the core wire 14 and is generally positioned distally from the proximal cylindrical member 16. The intermediate cylindrical member 38 is spaced apart from the proximal cylindrical member 16 such that the space between the proximal and intermediate cylindrical members 16, 38 forms a first gap 40.

A distal cylindrical member 18 is disposed about the elongated core wire 14 and is generally positioned distally from the intermediate cylindrical member 38. The distal cylindrical member 18 is spaced apart from the intermediate cylindrical member 38 such that the space between the intermediate and distal cylindrical members 38, 18 forms a second gap 42. Preferably, the distal cylindrical member 18 is a helically wound flexible coil made from metal, but may alternatively be formed of a polymer material.

Mounted on the intermediate cylindrical member 38, the self-expanding stent 20 may take on many different patterns or configurations. Examples of such stents are disclosed in two U.S. Patent Applications, both entitled "Intravascular Stent Device," filed June 5, 2002, and having U.S. Serial Nos. 10/163,116 and 10/163,248 and assigned to the same assignee as the present patent application. Preferably, the stent 20 is coated with an agent, such as heparin or rapamycin, to prevent stenosis or restenosis of the vessel. Examples of such coatings are disclosed in U.S. Patent Nos. 5,288,711; 5,516,781; 5,563,146 and 5,646,160.

The self-expanding stent 20 is preferably laser cut from a tubular piece of nitinol to form a skeletal tubular member. The skeletal tubular member has a small diameter and a thin wall comprised of a plurality of cells which are formed by a plurality of interconnected strut members. Then, the nitinol is treated so as to exhibit superelastic properties at body temperature. Additionally, the stent 20 includes proximal and distal strut members 44, 46 coupled to the proximal and distal sections 48, 50 of the stent. Preferably, the proximal and distal strut members 44, 46 are cut to form threads on the strut members during the laser-cutting of the stent 20 from the tubular piece of nitinol. Radiopaque coils are then wound onto the threads of the proximal and distal strut members 44, 46 to form anchor members 52. Preferably, the stent 20 includes eight anchor members 52. When the self-expanding stent 20 is mounted on the elongated core wire 14, the anchor members 52 align with and are disposed within the first and second gaps 40, 42 thus engaging the stent with the elongated core wire. In this configuration, the stent 20 can be moved distally and proximally through the delivery lumen 7 of the balloon catheter 2. The self-expanding stent 20 is described in more detail in U.S. Patent Application, entitled "Expandable Stent with Radiopaque Markers and Stent Delivery System," filed on June 27, 2003 (Attorney Docket No. CRD-5001-US-CIP) and assigned to the same assignee as the present patent application.

Attached to the distal end 22 of the elongated core wire 14 is the capture basket 24. The capture basket 24 is spaced apart from the self-expanding stent 20. The distance between the proximal end of the capture basket 24 and the distal end of the self-expanding stent 20 is in a range of about one millimeter to two centimeters, but preferably in a range of about five millimeters to fifteen millimeters. The capture basket 24 is preferably comprised of a self-expanding metallic frame 54 and a mesh body 56. The metallic frame 54 is designed to collapse within the delivery lumen 7 of the balloon catheter 2, yet be capable of expanding and covering a blood vessel upon deployment. The mesh body 56 is intended to capture any embolic debris released during angioplasty of the blood vessel and the deployment of the self-expanding stent 20 within the blood vessel.

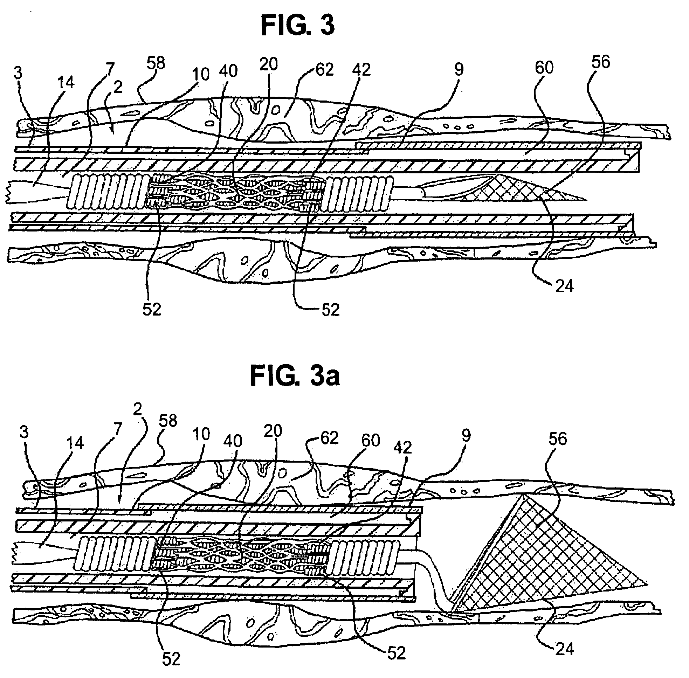

Figure 3 shows the balloon catheter 2 inserted within a blood vessel 58 of the brain of a patient. The balloon catheter 2 includes an expandable balloon 9 disposed about the distal portion 10 of the elongated outer catheter 3. In the preferred embodiment of the present invention, an inflation lumen 60 extends from the activation port 8 and communicates with the balloon 9. To perform an angioplasty of the blood vessel 58, a fluid is injected into the inflation lumen 60, through the activation port 8, to thus expand the balloon 9. The balloon catheter 2 is described in more detail in U.S. Patent No. 6,585,687, entitled "Inflatable Balloon Catheter Body Construction," assigned to the same assignee as the present patent application.

Typically, the balloon catheter 2 is advanced distally through the blood vessel 58 over a guidewire until it is aligned with a stenosis 60. Then, the guidewire is removed and the elongated core wire 14 is inserted into the delivery lumen 7 of the balloon catheter 2. The self-expanding stent 20 is mounted on the elongated core wire 14 such that the anchor members 52 align with and are disposed within the first gap 40, between the proximal and intermediate cylindrical members 16, 38, and the second gap 42, between the intermediate and distal cylindrical members 38, 18. In this configuration, the stent 20 is engaged to the core wire 14 so that the stent may be moved proximally and distally through the delivery lumen 7 of the balloon catheter 2.

As shown in Figure 3a, the elongated core wire 14 is advanced distally through the delivery lumen 7 of the balloon catheter 2 until the capture basket 24 has exited the delivery lumen and fully expanded within the blood vessel 58 distal of the stenosis 62. With the capture basket 24 fully deployed within the blood vessel 58, any embolic debris released from the stenosis 62 will be captured within the mesh body 56 of the capture basket, and thus removed from the blood vessel after the completion of the procedure.

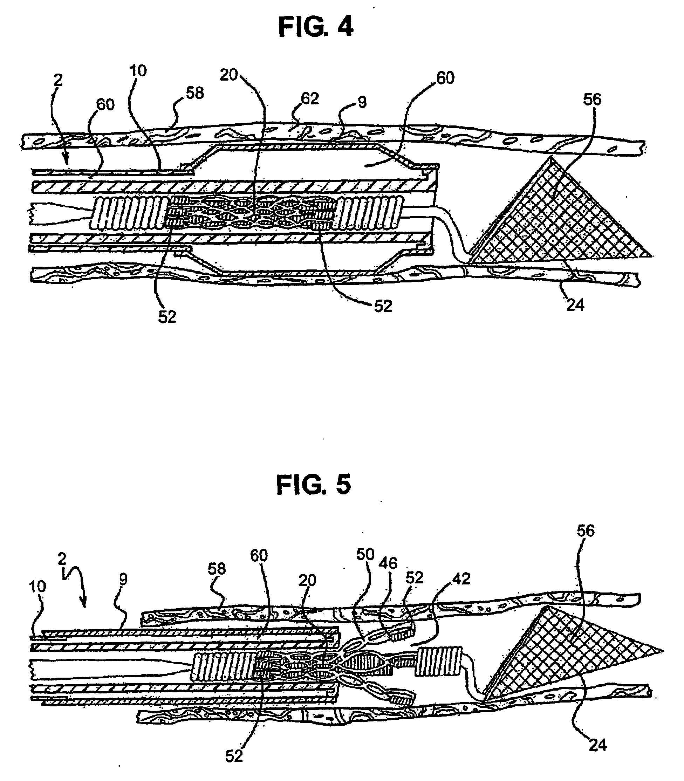

Figure 4 illustrates the balloon catheter 2 having the expandable balloon 9 fully expanded within the blood vessel 58. Preferably, the balloon 9 is expanded by injecting fluid into the inflation lumen 60 of the balloon catheter. The expanded balloon 9 compresses the stenosis 62 and thus increases the luminal diameter of the blood vessel 58. During the compression of the stenosis 62, embolic debris may dislodge from the stenosis and flow down the blood stream. In this case, the capture basket 24 will filter the blood and collect any embolic debris in the blood stream.

In Figure 5, the balloon 9 is contracted and the balloon catheter 2 is moved proximally, releasing anchor members 52 on the distal strut members 46 from the second gap 42 and allowing the distal section 50 of the self-expanding stent 20 to begin expanding. During expansion, the distal section 50 of the stent 20 comes in contact with the wall of the blood vessel 58.

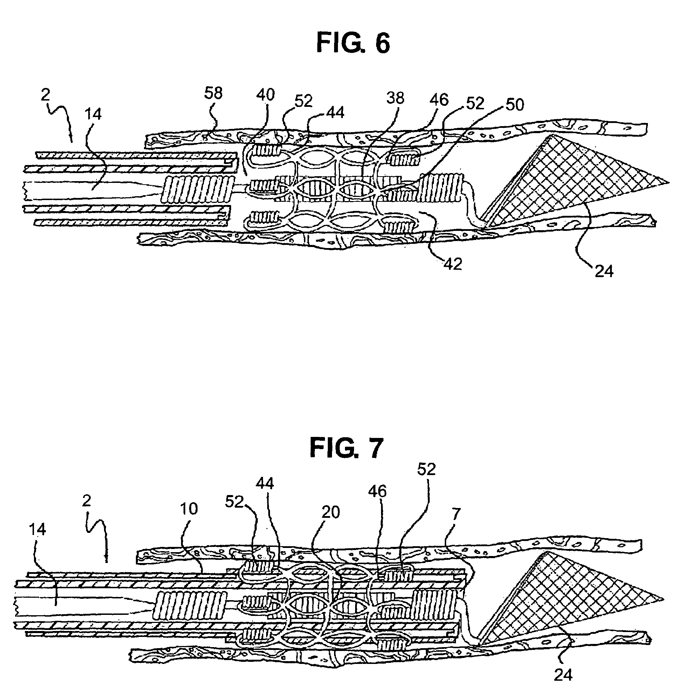

As illustrated in Figure 6, the balloon catheter 2 is again moved proximally, releasing the anchor members 52 on the proximal strut members 44 from the first gap 40 and allowing the proximal section 48 of the self-expanding stent 20 to expand. Once the stent 20 is fully deployed within the blood vessel 58, the core wire 14 remains extended through the stent 20 and thus serves as a guidewire, providing a physician with easier access to locations within the blood vessel distal of the stent.

If, during the deployment process, it is determined that the stent 20 should be relocated or realigned, the balloon catheter 2 may be used to resheath the stent 20. With the stent 20 mounted on the core wire 14 as described above, if the balloon catheter 2 is not withdrawn beyond the anchor members 52 on the proximal strut members 44, the stent will remain interlocked on the core wire 14. In this configuration, the stent 20 may be resheathed. To resheath the stent 20, the balloon catheter 2 is moved distally forcing the stent back onto the intermediate cylindrical member 38, compressing the distal section 50 of the stent, and forcing the anchor members 52 on the distal strut members 46 to become interlocked within the second gap 42. The stent 20 and balloon catheter 2 may then be withdrawn or repositioned to a different location within the blood vessel 58.

Figure 7 illustrates the balloon catheter 2 advanced distally over the elongated core wire 14 and through the self-expanding stent 20. The balloon catheter 2 is advanced distally until the capture basket 24 has collapsed within the delivery lumen 7 of the balloon catheter.

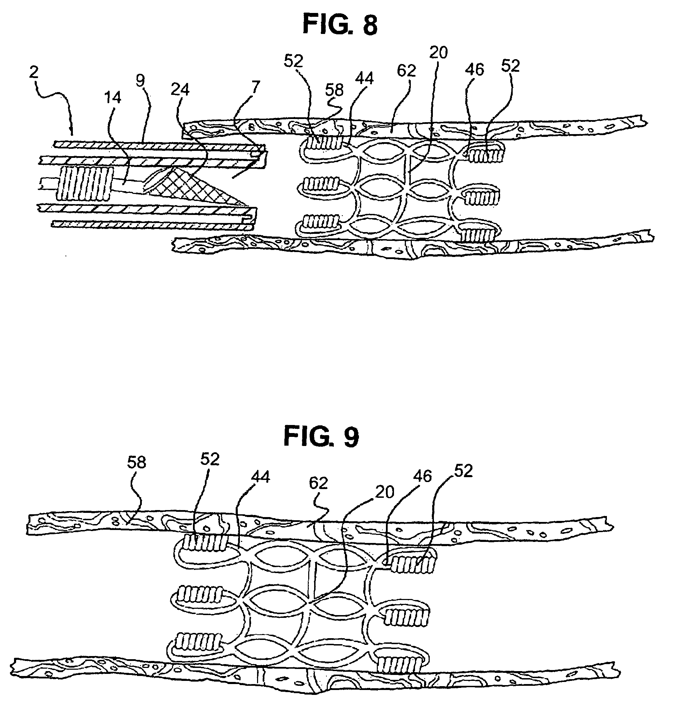

As shown in Figure 8, when the capture basket 24 collapses within the delivery lumen 7 of the balloon catheter 2, the balloon catheter and elongated core wire 14 may be removed from within the blood vessel 58. In this fashion, embolic debris captured within the capture basket 24 can be retained and removed from within the blood vessel. The capture basket thus prevents dislodgements from the preceding procedure to travel down the blood stream and create further complications such as ischemic strokes.

Figure 9 shows the self-expanding stent 20 fully expanded within the blood vessel 58 with the balloon catheter 2 removed from within the blood vessel. The stent 20 compresses the stenosis 62 and thus aids in preventing restenosis.

A novel system has been disclosed in which a self-expanding stent is mounted on an elongated core member and is slidably disposed within a balloon catheter. Although a preferred embodiment of the present invention has been described, it is to be understood that various modifications may be made by those skilled in the art without departing from the scope of the claims which follow.

## Claims

1. A self-expanding stent and stent delivery system comprising:
a balloon catheter comprised of an elongated catheter having a delivery lumen and an inflation lumen, said elongated catheter having a distal section and an inflatable balloon mounted on the distal section of said elongated catheter and communicating with the inflation lumen;
an elongated core member slidably disposed within the delivery lumen of said elongated catheter, said elongated core member including a proximal cylindrical member disposed about said elongated core member, and a distal cylindrical member disposed about said elongated core member and positioned distally of said proximal cylindrical member and being spaced apart from said proximal cylindrical member to define a gap having a predetermined length; and,
a self-expanding stent comprised of a small diameter skeletal tubular member having a thin wall, said wall of said skeletal tubular member including a plurality of cells which are formed by a plurality of interconnected strut members, and a cylindrical anchor member placed on one of said plurality of strut members, said cylindrical anchor member having a length less than the length of the gap between the proximal cylindrical member and the distal cylindrical member, and said self-expanding stent being mounted on one of said cylindrical members such that said cylindrical anchor member is interlocked within said gap and between said proximal cylindrical member and said distal cylindrical member to thereby retain said stent on said elongated core member.

2. A self-expanding stent and stent delivery system as defined in Claim 1, wherein said elongated core member takes the form of a wire.

3. A self-expanding stent and stent delivery system as defined in Claim 1 or Claim 2, wherein said skeletal tubular member includes threads formed on one of said plurality of strut members, and wherein said cylindrical anchor member takes the form of a helically wound coil, formed of radiopaque material, wound onto said threads.

4. A self-expanding stent and stent delivery system as defined in Claim 1 or Claim 2, wherein said self-expanding stent includes eight cylindrical anchor members, each cylindrical anchor member taking the form of a helically wound coil, formed of radiopaque material, wound onto threads which are formed on each one of said eight cylindrical anchor members.

5. A self-expanding stent and stent delivery system comprising:
a balloon catheter comprised of an elongated catheter having a delivery lumen, said elongated catheter having a distal section and an expandable balloon mounted on the distal section of said elongated catheter;
an elongated core member slidably disposed within the delivery lumen of said elongated catheter and having a stop member extending radially outward from said elongated core member; and,
a self-expanding stent mounted on said elongated core member and engaging said stop member so that said self-expanding stent can be moved through said delivery lumen when said elongated core member is moved through said delivery lumen.

6. A self-expanding stent and stent delivery system as defined in Claim 5, wherein said elongated core member takes the form of a wire.

7. A self-expanding stent and stent delivery system as defined in Claim 6, wherein said balloon catheter includes an inflation lumen communicating with said expandable balloon.

8. A self-expanding stent and stent delivery system as defined in Claim 6 or Claim 7, wherein said stop member takes the form of a cylindrical coil disposed about said elongated core member.

9. A self-expanding stent and stent delivery system as defined in any one of Claims 6 to 8, wherein said self-expanding stent is comprised of a small diameter skeletal tubular member having a thin wall, said wall of said skeletal tubular member including a plurality of cells which are formed by a plurality of interconnected strut members, and a cylindrical anchor member disposed about one of said plurality of strut members.
